(19) **Europäisches Patentamt / European Patent Office / Office européen des brevets**

(11) **EP 1 416 917 B1**

(12) **EUROPEAN PATENT SPECIFICATION**

(45) Date of publication and mention
of the grant of the patent:
**27.06.2007 Bulletin 2007/26**

(21) Application number: **02745697.9**

(22) Date of filing: **01.08.2002**

(51) Int Cl.:
*A61K 9/10* (2006.01)     *A61K 9/16* (2006.01)

(86) International application number:
**PCT/GB2002/003583**

(87) International publication number:
**WO 2003/013472 (20.02.2003 Gazette 2003/08)**

(54) **AQUEOUS DISPERSION COMPRISING STABLE NANOPARTICLES OF A WATER-INSOLUBLE ACTIVE AND AN EXCIPIENT LIKE MIDDLE CHAIN TRIGLYCERIDES (MCT)**

WÄSSRIGE DISPERSION STABILER NANOPARTIKEL EINES WASSERUNLÖSLICHEN WIRKSTOFFS UND EIN HILFSSTOFF WIE MITTELKETTIGE TRIGLYCERIDE (MCT)

DISPERSION AQUEUSE COMPRENANT DES NANOPARTICULES STABLES D'UN TRIGLYCERIDE A CHAINE MOYENNE (MTC) INSOLUBLE DANS L'EAU ACTIF ET D'UN TRIGLYCERIDE A CHAINE MOYENNE DE TYPE EXCIPIENT

(84) Designated Contracting States:
**AT BE BG CH CY CZ DE DK EE ES FI FR GB GR IE IT LI LU MC NL PT SE SK TR**
Designated Extension States:
**RO SI**

(30) Priority: **06.08.2001 GB 0119081**
**30.05.2002 GB 0212463**

(43) Date of publication of application:
**12.05.2004 Bulletin 2004/20**

(73) Proprietor: **AstraZeneca AB**
**151 85 Södertälje (SE)**

(72) Inventors:
 • **SKANTZE, Pia, Margaretha, Cecilia**
 **S-431 83 Molndal (SE)**
 • **SKANTZE, Tommy, Urban**
 **S-431 83 Molndal (SE)**
 • **VON CORSWANT, Lars, Christian**
 **S-431 83 Molndal (SE)**

 • **ZZACKRISSON SCHANTZ, Anna, Elisabeth**
 **Fysi**
 **S-412 96 Göteborg (SE)**
 • **LINDFORS, Per, Lennart**
 **S-431 83 Molndal (SE)**
 • **OLSSON, Ulf**
 **S-240 12 Torna Hällestad (SE)**

(74) Representative: **Nelson, Michael Andrew et al**
**AstraZeneca AB,**
**Global Intellectual Property**
**151 85 Sodertalje (SE)**

(56) References cited:
**US-A- 5 100 591**

 • **WELIN-BERGER K ET AL: "Inhibition of Ostwald ripening in local anesthetic emulsions by using hydrophobic excipients in the disperse phase." INTERNATIONAL JOURNAL OF PHARMACEUTICS. NETHERLANDS 10 MAY 2000, vol. 200, no. 2, 10 May 2000 (2000-05-10), pages 249-260, XP002225985 ISSN: 0378-5173 cited in the application**

EP 1 416 917 B1

**Description**

The present invention relates to a process for the preparation of a stable dispersion of particles, particularly sub-micron particles in an aqueous medium and to a stable dispersion of particles in a liquid medium, more particularly to a process for the preparation of a dispersion of particles comprising a substantially water-insoluble pharmacologically active compound in an aqueous medium, which particles exhibit substantially no increase in size upon storage in the aqueous medium, in particular to aqueous dispersions of particles that exhibit substantially no particle growth mediated by Ostwald ripening.

Dispersions of a solid material in a liquid medium are required for a number of different applications including paints, inks, dispersions of pesticides and other agrochemicals, dispersions of biocides and dispersions of pharmacologically active compounds. In the pharmaceutical field many pharmacologically active compounds have very low aqueous solubility which can result in low bioavailability when such compounds are administered to a patient. The bioavailability of such compounds may be improved by reducing the particle size of the compound, particularly to a sub-micron size, because this improves dissolution rate and hence absorption of the compound.

The formulation of a pharmacologically active compound as an aqueous suspension, particularly a suspension with a sub-micron particle size, enables the compound to be administered intravenously thereby providing an alternative route of administration which may increase bioavailability compared to oral administration.

Generally however, if there is a range of particles sizes dispersed in a medium there will be a differential rate of dissolution of the particles in the medium. The differential dissolution results in the smaller particles being thermodynamically unstable relative to the larger particles and gives rise to a flux of material from the smaller particles to the larger particles. The effect of this is that the smaller particles dissolve in the medium, whilst material is deposited onto the larger particles thereby giving an increase in particle size. One such mechanism for particle growth is known as Ostwald ripening (Ostwald, Z Phys. Chem. (34), 1900,495-503).

The growth of particles in a dispersion can result in instability of the dispersion during storage resulting in the sedimentation of particles from the dispersion. It is particularly important that the particle size in a dispersion of a pharmacologically active compound remains constant because a change in particle size is likely to affect the bioavailability and hence the efficacy of the compound. Furthermore, if the dispersion is required for intravenous administration, growth of the particles in the dispersion may render the dispersion unsuitable for this purpose, possibly leading to adverse or dangerous side effects.

Theoretically particle growth resulting from Ostwald ripening would be eliminated if all the particles in the dispersion were the same size. However, in practice, it is not possible to achieve a completely uniform particle size and even small differences in particle sizes can give rise to particle growth.

Aqueous suspensions of a solid material can be prepared by mechanical fragmentation, for example by milling. US 5,145,648 describes wet milling of a suspension of a sparingly soluble compound in an aqueous medium. However, mechanical fragmentation of a material, for example by milling, generally gives a wide distribution of particle sizes. Furthermore, mechanical fragmentation is less efficient in terms of particle size reduction when applied to non-crystalline starting material.

US 4,826,689 describes a processes for the preparation of uniform sized particles of a solid by infusing an aqueous precipitating liquid into a solution of the solid in an organic liquid under controlled conditions of temperature and infusion rate, thereby controlling the particle size. US 4,997,454 describes a similar process in which the precipitating liquid is non-aqueous. However, when the particles have a small but finite solubility in the precipitating medium particle size growth is observed after the particles have been precipitated. To maintain a particular particle size using these processes it is necessary to isolate the particles as soon as they have been precipitated to minimise particle growth. Therefore, particles prepared according to these processes cannot be stored in a liquid medium as a dispersion. Furthermore, for some materials the rate of Ostwald ripening is so great that it is not practical to isolate small particles (especially nano-particles) from the suspension.

W. J. Higuchi and J. Misra (J. Pharm. Sci., 51 (1962) 459) describe a method for inhibiting the growth of the oil droplets in oil-in-water emulsions by adding a hydrophobic compound (such as hexadecane) to the oil phase of the emulsion. US 6,074,986 (WO95/07614) describes the addition of a polymeric material having a molecular weight of up to 10,000 to the disperse oil phase of an oil-in-water emulsion to inhibit Ostwald ripening. Welin-Berger et al. (Int. Jour. of Pharmaceutics 200 (2000) pp 249-260) describe the addition of a hydrophobic material to the oil phase of an oil-in-water emulsion to inhibit Ostwald ripening of the oil droplets in the emulsion. In these latter three references the material added to the oil phase is dissolved in the oil phase to give a single phase oil dispersed in the aqueous continuous medium.

EP 589 838 describes the addition of a polymeric stabilizer to stabilize an oil-in-water emulsion wherein the disperse phase is a hydrophobic pesticide dissolved in a hydrophobic solvent.

US 4,348,385 discloses a dispersion of a solid pesticide in an organic solvent to which is added an ionic dispersant to control Ostwald ripening.

WO 99/04766 describes a process for preparing vesicular nano-capsules by forming an oil-in-water emulsion wherein the dispersed oil phase comprises a material designed to form a nano-capsule envelope, an organic solvent and optionally an active ingredient. After formation of a stable emulsion the solvent is extracted to leave a dispersion of nano-capsules.

US 5,100,591 describes a process in which particles comprising a complex between a water insoluble substance and a phospholipid are prepared by co-precipitation of the substance and phospholipid into an aqueous medium. Generally the molar ratio of phospholipid to substance is 1:1 to ensure that a complex is formed.

US 4,610,868 describes lipid matrix carriers in which particles of a substance is dispersed in a lipid matrix. The major phase of the lipid matrix carrier comprises a hydrophobic lipid material such as a phospholipid.

We have surprisingly found that stable dispersions of solid particles in an aqueous medium can be prepared using a precipitation process without the need for water-immiscible solvents or the formation of an emulsion. The dispersions prepared according to the present invention exhibit little or no particle growth after precipitation mediated by Ostwald ripening.

According to a first aspect of the present invention there is provided a process for the preparation of a stable dispersion of solid particles in an aqueous medium comprising:

combining (a) a first solution comprising a pharmacologically active substance having a solubility in water at 25°C of less than 0,5 mg/ml, a water-miscible organic solvent and an inhibitor with (b) an aqueous phase comprising water and optionally a stabiliser, thereby precipitating solid particles comprising the inhibitor and the pharmacologically active substance; and optionally removing the water-miscible organic solvent; wherein:

(i) the inhibitor is a non-polymeric hydrophobic organic compound that is substantially insoluble in water;
(ii) the inhibitor is less soluble in water than the pharmacologically active substance;
(iii) the inhibitor is not a phospholipid and (iv) the inhibitor is sufficiently miscible with the pharmacologically active substance to form said particles in the dispersion comprising a substantially single phase mixture of the substance and the inhibitor.

The process according to the present invention enables stable dispersions of very small particles, especially nano-particles, to be prepared in high concentration without the need to quickly isolate the particles from the liquid medium into which they have been precipitated to prevent particle growth.

The dispersion according to the present invention is stable, by which we mean that the solid particles in the dispersion exhibit reduced or substantially no particle growth mediated by Ostwald ripening. By the term "reduced particle growth" is meant that the rate of particle growth mediated by Ostwald ripening is reduced compared to particles prepared without the use of an inhibitor. By the term "substantialy no particle growth" is meant that the mean particle size of the particles in the aqueous medium does not increase by more than 10% (more preferably by not more than 5%) over a period of 1 hour at 20°C after precipitation into the aqueous phase in the present process. Preferably the particles exhibit substantially no particle growth.

It is to be understood that in those cases where the solid particles are precipitated in an amorphous form the resulting particles will, generally, eventually revert to a thermodynamically more stable crystalline form upon storage as an aqueous dispersion. The time taken for such dispersions to re-crystallise is dependent upon the substance and may vary from a few hours to a number of days. Generally such re-crystallisation will result in particle growth and the formation of large crystalline particles which are prone to sedimentation from the dispersion. It is to be understood that the present invention does not prevent conversion of amorphous particles in the suspension into a crystalline state. The presence of the inhibitor in the particles according to the present invention significantly reduces or eliminates particle growth mediated by Ostwald ripening, as hereinbefore described. The particles are therefore stable to Ostwald ripening and the tem "stable" used herein is to be construed accordingly.

The solid particles in the dispersion preferably have a mean particle size of less than 10μm, more preferably less than 5μm, still more preferably less than 1μm and especially less than 500nm. It is especially preferred that the particles in the dispersion have a mean particle size of from 10 to 500nm, more especially from 50 to 300nm and still more especially from 100 to 200nm. The mean size of the particles in the dispersion may be measured using conventional techniques, for example by dynamic light scattering to measure the intensity-averaged particle size.

Generally the solid particles in the dispersion prepared according to the present invention exhibit a narrow unimodal particle size distribution.

The solid particles may be crystalline, semi-crystalline or amorphous. In an embodiment, the solid particles comprise a pharmacologically active substance in a substantially amorphous form. This can be advantageous as many pharmacological compounds exhibit increased bioavailability in amorphous form compared to their crystalline or semi-crystalline forms. The precise form of the particles obtained will depend upon the conditions used during the precipitation step of the process. Generally, the present process results in rapid precipitation of the substance and the formation of substantially amorphous particles.

The pharmacologically active substance in the first solution has a solubility in water at 25°C of less than 0.5mg/ml, preferably less than 0.1mg/ml and especially less than 0.05mg/ml.

The greatest effect on particle growth inhibition is observed when the substance has a solubility in water at 25°C of mono than $0.05\mu g/ml$. In a preferred embodiment the substance has a solubility in the range of from $0.05\mu g/ml$ to 0.5mg/ml, for example from $0.05\mu g/ml$ to 0.05mg/ml.

The solubility of the substance in water may be measured using a conventional technique. For example, a saturated solution of the substance is prepared by adding an excess amount of the substance to water at 25°C and allowing the solution to equilibrate for 48 hours. Excess solids are removed by centrifugation or filtration and the concentration of the substance in water is determined by a suitable analytical technique such as HPLC.

Numerous classes of pharmacologically active compounds are suitable for use in the present invention including but not limited to anti-cancer agents (for example bicalutamide), steroids, preferably glucocorticosteroids (especially auti-inflammatory glucocorticosteroids, for example budesonide) antihypertensive agents (for example felodipine or prazosin), beta-blockers (for example pindolol or propranolol), hypolipidaemic agents, aniticoagulants, antithrombotics, antifungal agents (for example griseofluvin), antiviral agents, antibiotics, antibacterial agents (for example ciprofloxacin), antipsychotic agents, antidepressants, sedatives, anaesthetics, anti-inflammatory agents (including compounds for the treatment of gastrointestinal inflammatory diseases, for example compounds described in WO99/55706 and other anti-inflammatory compounds, for example ketoprofen), antihistamines, hormones (for example testosterone), immunomodifiers, or contraceptive agents. The substance may comprise a single substance having a solubility in water at 25°C of less than 0.5 mg/ml or a combination of two or more such substances.

Inhibitor

The inhibitor is a non-polymeric hydrophobic organic compound that is less soluble in water than the pharmacologically active substance present in the first solution, and wherein the inhibitor is not a phospholipid. Suitable inhibitors have a water solubility at 25°C of less than 0.1mg/l, more preferably less than 0.01 mg/l. In an embodiment of the invention the inhibitor has a solubility in water at 25°C of less than $0.05\mu g/ml$, for example from 0.1ng/ml to $0.05\mu g/ml$.

In an embodiment of the invention the inhibitor has a molecular weight of less than 2000, such as less than 500, for example less than 400. In another embodiment of the invention the inhibitor has a molecular weight of less than 1000, for example less than 600. For example, the inhibitor may have a molecular weight in the range of from 200 to 2000, preferably a molecular weight in the range of from 400 to 1000, mom preferably from 400 to 600.

600. Suitable inhibitors include an inhibitor selected from classes (i) to (vi) or a combination of two or more such inhibitors:

(i) a mono-, di- or (more preferably) a tri-glyceride of a fatty acid. Suitable fatty acids include medium chain fatty acids containing from 8 to 12, more preferably from 8 to 10 carbon atoms or long chain fatty acids containing more than 12 carbon atoms, for example from 14 to 20 carbon atoms, more preferably from 14 to 18 carbon atoms. The fatty acid may be saturated, unsaturated or a mixture of saturated and unsaturated acids. The fatty acid may optionally contain one or more hydroxyl groups, for example ricinoleic acid. The glyceride may be prepared by well known techniques, for example, esterifying glycerol with one or more long or medium chain fatty acids. In a preferred embodiment the inhibitor is a mixture of triglycerides obtainable by esterifying glycerol with a mixture of long or, preferably, medium chain fatty acids. Mixtures of fatty acids may be obtained by extraction from natural products, for example from a natural oil such as palm oil. Fatty acids extracted from palm oil contain approximately 50 to 80% by weight decanoic acid and from 20 to 50% by weight of octanoic acid. The use of a mixture of fatty acids to esterify glycerol gives a mixture of glycerides containing a mixture of different acyl chain lengths. Long and medium chain triglycerides are commercially available. For example a preferred medium chain triglyceride (MCT) containing acyl groups with 8 to 12, more preferably 8 to 10 carbon atoms is prepared by esterification of glycerol with fatty acids extracted from palm oil, giving a mixture of triglycerides containing acyl groups with 8 to 12, more preferably 8 to 10 carbon atoms. This MCT is commercially available as Miglyol 812N (Huls, Germany). Other commercially available MCT's include Miglyol 810 and Miglyol 818 (Huls, Germany). A further suitable medium chain triglyceride is trilaurine (glycerol trilaurate). Commercially available long chain trigylcerides include soya bean oil, sesame oil, sunflower oil, castor oil or rape-seed oil.

Mono and di- glycerides may be obtained by partial esterification of glycerol with a suitable Fatty acid, or mixture of fatty acids. If necessary the mono- and di- glycerides may be separated and purified using conventional techniques, for example by extraction from a reaction mixture following esterification. When a mono-glyceride is used it is preferably a long-chain mono glyceride, for example a mono glyceride formed by esterification of glycerol with a fatty acid containing 18 carbon atoms;

(ii) a fatty acid mono- or (preferably) di-ester of a $C_{2-10}$ diol. Preferably the diol is an aliphatic diol which may be saturated or unsaturated, for example a $C_{2-10}$-alkane diol which may be a straight chain or branched chain diol. More preferably the diol is a $C_{2-6}$-alkane diol which may be a straight chain or branched chain, for example ethylene glycol or propylene glycol. Suitable fatty acids include medium and long chain fatty acids described above in relation to the glycerides. Preferred esters are di-esters of propylene glycol with one or more fatty acids containing from 8 to 10 carbon atoms, for example Miglyol 840 (Huls, Gepmany);

(iii) a fatty acid ester of an alkanol or a cycloalkanol. Suitable alkanols include $C_{1-10}$-alkanols, more preferably $C_{2-6}$-alkanols which may be straight chain or branched chain, for example ethanol, propanol, isopropanol, n-butanol, sec-butanol or tert-butanol. Suitable cycloalkanols include $C_{3-6}$-cycloalkanols, for example cyclohexanol. Suitable fatty acids include medium and long chain fatty acids described above in relation to the glycerides. Preferred esters are esters of a $C_{2-6}$-alkanol with one or more fatty acids containing from 8 to 10 carbon atoms, or more preferably 12 to 29 carbon atoms, which fatty acid may saturated or unsaturated. Suitable esters include, for example isopropyl myristate or ethyl oleate;

(iv) a wax. Suitable waxes include esters of a long chain fatty acid with an alcohol containing at least 12 carbon atoms. The alcohol may an aliphatic alcohol, an aromatic alcohol, an alcohol containing aliphatic and aromatic groups or a mixture of two or more such alcohols. When the alcohol is an aliphatic alcohol it may be saturated or unsaturated. The aliphatic alcohol may be straight chain, branched chain or cyclic. Suitable aliphatic alcohols include those containing more than 12 carbon atoms, preferably more than 14 carbon atoms especially more than 18 carbon atoms, for example from 12 to 40, more preferably 14 to 36 and especially from 18 to 34 carbon atoms. Suitable long chain fatty acids include those described above in relation to the glycerides, preferably those containing more than 14 carbon atoms especially more than 18 carbon atoms, for example from 14 to 40, more preferably 14 to 36 and especially from 18 to 34 carbon atoms. The wax may be a natural wax, for example bees wax, a wax derived from plant material, or a synthetic wax prepared by esterification of a fatty acid and a long chain alcohol. Other suitable waxes include petroleum waxes such as a paraffin wax;

(v) a long chain aliphatic alcohol. Suitable alcohols include those with 6 or more carbon atoms, more preferably 8 or more carbon atoms, such as 12 or more carbon atoms, for example from 12 to 30, for example from 14 to 20 carbon atoms. It is especially preferred that the long chain aliphatic alcohol has from 6 to 20, more especially from 6 to 14 carbon atoms, for example from 8 to 12 carbon atoms. The alcohol may be straight chain, branched chain, saturated or unsaturated. Examples of suitable long chain alcohols include, 1-hexanol, 1-decanol, 1-hexadecanol, I-octadecanol, or 1-heptadecanol (more preferably 1-decanol); or

(vi) a hydrogenated vegetable oil, for example hydrogenated castor oil.

In one embodiment of the present invention the inhibitor is selected from a medium chain triglyceride and a long chain aliphatic alcohol containing from 6 to 12, preferably from 10 to 20 carbon atoms. Preferred medium chain triglycerides and long chain aliphatic alcohols are as defined above, In a preferred embodiment the inhibitor is selected from a medium chain triglyceride containing acyl groups with from 8 to 12 carbon atoms or a mixture of such triglycerides (preferably Miglyol 812N) and an aliphatic alcohol containing from 10 to 14 carbon atoms (preferably 1-decanol) or a mixture thereof (for example a mixture comprising Miglyol 812N and 1-decanol).

Suitably the inhibitor is a liquid at the temperature at which the dispersion is prepared. Preferably the inhibitor is liquid at ambient temperature (25°C).

The inhibitor is preferably a pharmaceutically inert material.

The inhibitor is present in the particles in a quantity sufficient to prevent Ostwald ripening of the particles in the suspension. Preferably the inhibitor will be the minor component in the solid particles formed in the present process comprising the inhibitor and the pharmacologically active substance. Preferably, therefore, the inhibitor is present in a quantity that is just sufficient to prevent Ostwald ripening of the particles in the dispersion, thereby minimising the amount of inhibitor present in the particles.

In embodiments of the present invention the weight fraction of inhibitor relative to the total weight of inhibitor and pharmacologically active substance (i.e. weight of inhibitor/weight of inhibitor + weight of pharmacologically active substance) is from 0.01 to 0.99, preferably from 0.01 to 0.5, especially from 0.05 to 0.3 and more especially from 0.06 to 0.25. In a preferred embodiment the weight fraction of inhibitor relative to the total weight of inhibitor and pharmacologically active substance is less than 0.5, more preferably 0.3 or less, for example from 0.05 to 0.3. such as from 0.06 to 0.25, for example about 0.2. This is particularly preferred for a pharmacologically active substance as a high level of inhibitor (e.g. a weight fraction above 0.5) may give rise to unwanted side effects and/or affect the dissolution rate/bioavailability of the pharmacologically active substance when administered *in vivo.*

Furthermore, we have found that in general a low weight ratio of inhibitor to the inhibitor and the pharmacologically active substance (i.e. less than 0.5) is sufficient to prevent particle growth by Ostwald ripening, thereby allowing small (preferably less than 1μm, preferably less than 500nm) stable particles to be prepared. A small and constant particle size is often desirable, especially when the pharmacologically active substance is used, for example, for intravenous administration.

One application of the dispersions prepared by the process according to the present invention is the study of the toxicology of a pharmacologically active compound. The dispersions prepared according to the present process can exhibit improved bioavailability compared to dispersions prepared using alternative processes, particularly when the particle size of the substance is less than $0.5\mu$m. In this application it is advantageous to minimise the amount of inhibitor relative to the active compound so that any effects on the toxicology. associated with the presence of the inhibitor are minimised.

When the pharmacologically active substance has an appreciable solubility in the inhibitor the weight ratio of inhibitor to pharmacologically active substance should be selected to ensure that the amount of pharmacologically active substance exceeds that requited to form a saturated solution of the pharmacologically active substance in the inhibitor. This ensures that solid particles of the pharmacologically active substance are formed in the dispersion. This is important when the inhibitor is a liquid at the temperature at which the dispersion is prepared (for example ambient temperature) to ensure that the process does not result in the formation liquid droplets comprising a solution of the pharmacologically active substance in the inhibitor, or a two phase system comprising the solid substance and large regions of the liquid inhibitor.

Without wishing to be bound by theory we believe that systems in which there is a phase separation between the substance and inhibitor in the particles are more prone to Ostwald ripening than those in which the solid particles form a substantially single phase system. Accordingly, in a preferred embodiment the inhibitor is sufficiently miscible in the pharmacologically active material to form solid particles in the dispersion comprising a substantially single-phase mixture of the substance and the inhibitor. The composition of the particles formed according to the present invention may be analysed using conventional techniques, for example analysis of the (thermodynamic) solubility of the pharmacologically active substance in the inhibitor, melting entropy and melting points obtained using routine differential scanning calorimetry (DSC) techniques to thereby detect phase separation in the solid particles. Furthermore, studies of nanosuspensions using nuclear magnetic resonance (NMR) (e.g. line broadening of either component in the particles) may be used to detect phase separation in the particles.

Generally the inhibitor should have a sufficient miscibility with the substance to form a substantially single phase particle, by which is meant that the inhibitor is molecularly dispersed in the solid particle or is present in small domains of inhibitor dispersed throughout the solid particle. It is thought that for many substances the substance/inhibitor mixture is a non-ideal mixture by which is meant that the mixing of two components is accompanied by a non-zero enthalpy change.

An indication of the substance/inhibitor miscibility in the solid particles is provided by the interaction parameter $\chi$ for the substance-inhibitor mixture. The $\chi$ parameter may be derived from the well known Bragg-Williams, Flory-Huggins or the Regular Solution theories (see e.g.. Jönsson, B. Lindman, K. Holmberg, B. Kronberg, "Surfactants and Polymers in Solution", John Wiley & Sons, 1998 and Neau et al, Pharmaceutical Research, 14, 601 1997). In an ideal mixture $\chi$ is 0, and according to the Bragg-Williams theory a two-component mixture will not phase separate provided $\chi<2$. We believe that in many particles prepared according to the present invention the substance and inhibitor are not ideal mixtures and therefore the $\chi$ value is not zero.

We have surprisingly found that when $\chi$ is < 2.5 the solid particles prepared according to the invention exhibit little or no Ostwald ripening. Those systems in which $\chi$ is >2.5 are thought to be prone to phase separation and are less stable to Ostwald ripening. Suitably the $\chi$ value of the substance-inhibitor mixture is 2 or less, for example from0 to 2, preferably 0.1 to 2, such as 0.2 to 1.8.

Many small molecule organic substances (Mw < 1000) are available in a crystalline form or can be prepared in crystalline form using conventional techniques (for example by recrystallisation from a suitable solvent system). In such cases the $\chi$ parameter of the substance and inhibitor mixture is easily determined from the Equation I:

$$\chi = \frac{-\Delta S_m \ln[T_m/T]/R - \ln x^s_1}{(1 - x^s_1)^2}$$

wherein:

$\Delta S_m$ is the entropy of melting of the crystalline pharmacologically active substance (measured using a conventional technique such as DSC measurement);

$T_m$ is the melting point (K) of the crystalline pharmacologically active substance (measured using a conventional technique such as DSC measurement);

T is the temperature of the dispersion (K);

R is the gas constant; and

$x^s_1$ is the mole fraction solubility of the crystalline pharmacologically active substance in the inhibitor (measured using conventional techniques for determining solubility for example as hereinbefore described). In the above equation $T_m$ and $\Delta S_m$ refer to the melting point of the crystalline form of the material. In those cases where the substance may exist in the form of different polymorphs, $T_m$ and $\Delta S_m$ are determined for the polymorphic form of the substance that is most stable at the temperature of the dispersion. As will be understood, the measurement of $\Delta S_m$. and $x^s_1$ are performed on the crystalline pharmacologically active substance prior to formation of the dispersion according to the invention and thereby enables a preferred inhibitor for the pharmacologically active material to be selected by performing simple measurements on the bulk crystalline material.

The mole fraction solubility of the crystalline pharmacologically active substance in the inhibitor ($x^s_1$) is simply the number of moles of substance per mole of inhibitor present in a saturated solution of the substance in the inhibitor. As will be realized the equation above is derived for a two component system of a substance and an inhibitor. In those systems where the inhibitor contains more than one compound (for example in the case of a medium chain triglyceride comprising a mixture of triglycerides such as Miglyol 812N, or where a mixture of inhibitors is used) it is sufficient to calculate $x^s_1$ in terms of the "apparent molarity" of the mixture of inhibitors. The apparent molarity of such a mixture is calculated for a mixture of n inhibitor components to be:

$$\text{Apparent molarity} = \left(\underline{\text{Mass of 1 litre of inhibitor mixture}}\right) * \left[\left(a/Mw_a\right) + \left(b/Mw_b\right) + \ldots \left(n/Mw_n\right)\right]$$

wherein: a, b .. n are the weight fraction of each component in the inhibitor mixture (for example for component a this is %w/w component a/100); and
Mwa....Mwn is the molecular weight of each component a..n in the mixture.

$x^s_1$ is then calculated as:

$$x^s_1 = \frac{\underline{\text{Molar solubility of the crystalline substance in the inhibitor mixture (mol/l)}}}{\text{Apparent molarity of inhibitor mixture (mol/l)}}$$

When the inhibitor is a solid at the temperature that the dispersion is prepared, the mole fraction solubility, $x^s_1$, can be estimated by measuring the mole fraction solubility at a series of temperatures above the melting point of the inhibitor and extrapolating the solubility back to the desired temperature. However, as hereinbefore mentioned, it is preferred that the inhibitor is a liquid at the temperature that the dispersion is prepared. This is advantageous because, amongst other things, the use of a liquid inhibitor enables the value of $x^s_1$ to be measured directly.

In certain cases, it may not be possible to obtain the pharmacologically active material in a crystalline form, particularly in the case of large organic molecules which are often amorphous. In such cases, preferred inhibitors are those which sufficiently miscible with the pharmacologically active material to form a substantially single phase mixture pharmacologically active material may be determined using routine experimentation. For example the substance and inhibitor may be dissolved in a suitable organic solvent followed by removal of the solvent to leave a mixture of the substance and inhibitor. The resulting mixture may then be characterised using a routine technique such as DSC characterisation to determine whether or not the mixture is a single phase system. This empirical method enables preferred inhibitors for a particular substance to be selected and will provide substantially single phase solid particles in the dispersion prepared according to the present invention.

In a further embodiment of the present invention the miscibility of the substance and the inhibitor may be increased by the addition of a suitable co-inhibitor to the first solution in the present process. The presence of the co-inhibitor increases the miscibility of the substance and the inhibitor mixture, thereby reducing the $\chi$ value and further reducing or preventing Ostwald ripening. Suitable co-inhibitors include an inhibitor as hereinbefore defined, preferably an inhibitor selected from classes (i) to (vi) listed hereinbefore. In a preferred embodiment when the inhibitor is a medium chain triglyceride containing acyl groups with 8 to 12 carbon atoms (or a mixture of such triglycerides such as Miglyol 812N), a preferred co-inhibitor is a long chain aliphatic alcohol containing 6 or more carbon atoms (preferably from 6 to 14 carbon atoms) for example 1-hexanol or more preferably 1-decanol. The weight ratio of inhibitonco-inhibitor is selected to give the desired $\chi$ value of the substance/inhibitor/co-inhibitor mixture and may be varied over wide limits, for example from 10:1 to 1:10, such as approximately 1:1. Preferred values for $\chi$ are as hereinbefore defined.

The inhibitor in the present invention is not a phospholipid. Such lipids have a hydrophilic phosphorous containing "head" groups and one or more lipophilic "tail" groups. Such phosphlipids are capable of forming lipid bilayers and exhibit surface-active effects. Examples of phospholipids excluded from the present invention include, for example the phospholipids described in US 5,100,591.

Water-Miscible Organic Solvent

The water-miscible organic solvent in the first phase is preferably miscible with water in all proportions. The water-miscible organic solvent should also be a solvent for both the pharmacologically active substance and the inhibitor. The water-miscible organic solvent is selected such that the inhibitor and the pharmacologically active substance each have a sufficient solubility in the water miscible organic solvent to enable a precipitate of the pharmacologically active substance to form when the first solution is combined with the aqueous phase. Suitably, the inhibitor and the pharmacologically active substance each have a solubility of 10mg/ml or more in the water-miscible organic solvent.

Generally it is preferred that the concentration of the pharmacologically active substance in the water-miscible organic solvent is as high as possible to aid efficient precipitation. The upper concentration of the pharmacologically active substance in the water-miscible organic solvent is determined by the solubility of the substance in the solvent. However, we have found that a wide range of concentrations may be used in the present process. Typically, a concentration of pharmacologically active substance of 1% by weight or more in the organic solvent is sufficient.

In the first solution the inhibitor and/or the pharmacologically active substance should be completely dissolved in the water-miscible organic solvent. The presence of particles of the inhibitor and/or the pharmacologically active substance in the first solution may result in poor control of the particle size distribution in the dispersion.

If required the solubility of the inhibitor and/or the pharmacologically active substance in the water-miscible organic solvent can be increased by heating a mixture of the inhibitor, pharmacologically active substance and water-miscible organic solvent to provide a solution. The solution is then maintained at elevated temperature until it is combined with the aqueous phase in the process.

As will be understood, the selection of water-miscible organic solvent will be dependent upon the nature of the pharmacologically active substance. When the pharmacologically active substance is an organic compound the water-miscible organic solvent should have a sufficiently low dielectric constant to be able to dissolve the pharmacologically active substance and the inhibitor. Suitable water-miscible solvents for dissolving a pharmacologically active organic substance include, a water-miscible alcohol, for example methanol, ethanol, n-propyl alcohol, isopropyl alcohol, tert-butyl alcohol, ethylene glycol or propylene glycol; dimethylsulfoxide; dimethylformamide; a water-miscible ether, for example tetrahydrofuran; a water-miscible nitrile, for example acetonitrile; a water-miscible ketone, for example acetone or methyl ethyl ketone; an amide, for example dimethylacetamide or a mixture of two or more of the above mentioned water-miscible organic solvents. A preferred water-miscible organic solvent is dimethylacetamide (DMA).

Precipitation

In the present process the first solution and the aqueous phase may be combined by adding the first solution to the aqueous phase. Alternatively, the aqueous phase may be added to the first solution. During the combination of the first solution and the aqueous phase the conditions are controlled to give precipitated solid particles of the required particle size. The particle size resulting from the combination of the first solution and aqueous phase is determined by a number of factors including, the rate of agitation during the combination of the first solution and the aqueous phase, the temperature during the combination and the rate at which the combination takes place. As will be clear, sufficient aqueous phase is used during the combination to extract sufficient water-miscible organic solvent from the first solution to cause precipitation of the solid particles from the first solution.

Suitable conditions for the addition of the aqueous phase to the first solution for the formation of sub-micron particles are described in US 4,826,689, incorporated herein by reference thereto, wherein an aqueous phase is injected into an agitated phase containing the substance dissolved in an organic solvent. Suitable rates of addition are typically from 100ml/min to 1000ml/min per 50ml of the first solution. A suitable temperature for the addition is from 0 to 100°C, more preferably from 5 to 50°C.

Addition of the aqueous phase into the first solution may be achieved using a number of techniques, for example by injecting the aqueous phase directly into the first solution (for example via a syringe) or by adding the aqueous phase drop-wise into the first solution. For larger scale production the aqueous phase may be added to the first solution using a flow mixer. Preferably the first solution is agitated during addition of the aqueous phase by for example stirring, preferably at a rate sufficient to induce a high degree of turbulence in the first solution and hence a very rapid precipitation and distribution of particles into the liquid medium of the dispersion. Alternatively, the first solution may be agitated by sonication in an ultrasonic bath.

When the first solution is added to the aqueous phase, the aqueous phase is preferably agitated as described above, thereby enhancing extraction of water-miscible solvent from the first solution to give small particles and good dispersion of the particles in the liquid medium. Suitable rates and methods of addition, temperature and degree of agitation are analogous to those described above for the addition of the aqueous phase into the first solution.

Some particles will precipitate and form a uniform dispersion without the need for a stabiliser in the aqueous phase. However, we have found that many particles tend to aggregate upon precipitation unless a stabiliser is present in the aqueous phase.

Stabilisers suitable for the prevention of particle aggregation in dispersions are well known to those skilled in the art. Suitable stabilisers include dispersants and surfactants (which may be anionic, cationic or non-ionic) or a combination thereof. Suitable dispersants include, a polymeric dispersant, for example a polyvinylpyrrolidone, a polyvinylalcohol or a cellulose derivative, for example hydroxypropylmethyl cellulose, hydroxy ethyl cellulose, ethylhydroxyethyl cellulose or carboxymethyl cellulose. Suitable anionic surfactants include alkyl and aryl sulphonates, sulphates or carboxylates, such as an alkali metal alkyl and aryl sulphonate or sulphate, for example, sodium dodecyl sulphate. Suitable cationic surfactants include quaternary ammonium compounds and fatty amines. Suitable non-ionic surfactants include, monoesters of sorbitan which may or may not contain a polyoxyethylene residue, ethers formed between fatty alcohols and polyoxyethylene glycols, polyoxyetheylene-polypropylene glycols, an ethoxylated castor oil (for example Cremophor EL), ethoxylated hydrogenated castor oil, ethoxylated 120H-stearic acid (for example Solutol HS15). The aqueous phase may contain a single stabiliser or a mixture of two or more stabilisers. In a preferred embodiment the aqueous phase contains a polymeric dispersant and a surfactant (preferably an anionic surfactant), for example a polyvinylpyrrolidone and sodium dodecyl sulphate. When the pharmacologically active material is a pharmacologically active compound it is preferred that the stabiliser is a pharmaceutically acceptable material.

Generally the aqueous phase will contain from 0.01 to 1% by weight, preferably from 0.05 to 0.5% by weight and especially from 0,1 to 0.2% by weight of stabiliser. We have found that the dispersions prepared according to the present process require lower levels of stabilisers (such as surfactants) compared to precipitation processes that do not use an inhibitor.

Optionally, additional stabiliser may be added to the dispersion after precipitation of the particles into the aqueous phase to provide additional inhibition of particle aggregation in the dispersion.

The combination of the first solution and aqueous phase in the process according to the present invention results in very fast, substantially instantaneous precipitation of particles of the inhibitor and pharmacologically active material to give particles of the desired size with a narrow particle size distribution. The precipitation avoids the need to form an emulsion prior to extraction of the water-miscible organic solvent, and thereby considerably simplifies the preparation of a dispersion of solid particles compared to emulsion-based processes.

Optionally the water-miscible organic solvent can be removed from the dispersion after the precipitation. Suitable methods for removing the water-miscible organic solvent include evaporation, for example by heating the dispersion under vacuum, reverse osmosis, dialysis, ultra-filtration or cross-flow filtration. The dispersion may be concentrated after precipitating the particles by removing excess water from the dispersion, for example by evaporation, spray drying or lyophilisation.

Optionally additional components may be added to the dispersion for example viscosity modifying agents, buffers, taste masking agents, anti-oxidants, preservatives or colorants. The additional components may be added before, or more preferably, after the precipitation of the particles.

According to a further embodiment of the present invention there is provided a process for the preparation of a stable dispersion of solid particles of a pharmacologically active substance having a solubility in water at 25°C of less than 0,5 mg/ml, in an aqueous medium comprising:

combining (a) a first solution comprising the pharmacologically active substance, a water-miscible organic solvent and an inhibitor with (b) an aqueous phase comprising water and optionally a stabiliser, thereby precipitating solid particles comprising the inhibitor and the pharmacologically active substance; and optionally removing the water-miscible organic solvent;

wherein the inhibitor is less soluble in water than the pharmacologically active substance, which inhibitor is selected from one or more of:

(i) a mono-, di- or (more preferably) a tri-glyceride of a fatty acid;
(ii) a fatty acid mono- or (preferably) di-ester of a $C_{2-10}$ diol;
(iii) a fatty acid ester of an alkanol or a cycloalkanol;
(iv) a wax;
(v) a long chain aliphatic alcohol (preferably containing 6 or more carbon atoms, for example from 8 to 12 carbon atoms); and
(vi) a hydrogenated vegetable oil.

This embodiment of the present invention provides stable dispersions of particles of a solid pharmacologically active substance in an aqueous medium. The dispersions prepared according to this embodiment exhibit little or no growth in particle size during storage (resulting from, Ostwald ripening).

In this embodiment it is preferred that the miscibility of the pharmacologically active substance and inhibitor are sufficient to give substantially single phase solid particles in the dispersion, more preferably the inhibitor/substance mixture has a $\chi$ value of <2.5, more preferably 2 or less, for example from 0 to 2, preferably from 0.1 to 2 wherein the $\chi$ value is as hereinbefore defined.

In this embodiment the inhibitor is preferably a medium chain tri-glyceride (MCT) containing acyl groups with 8 to 12 (more preferably 8 to 10) carbon atoms, or a mixture thereof, for example Miglyol 812N. The miscibility of the inhibitor with the substance may be increased by using a co-inhibitor as hereinbefore described. For example, a suitable inhibitor/co-inhibitar in this embodiment comprises a medium chain tri-glyceride (MCT) as defined above and a long chain aliphatic alcohol having 6 to 12 (more preferably 8 to 12, for example 10) carbon atoms, or a mixture comprising two or more such inhibitors (for example 1-hexanol or (more preferably) 1-deeaaol). A preferred inhibitor/co-inhibitor for use in this embodiment is a mixture of Miglyol 812N and 1-decanol.

If required the particles present in the dispersion prepared according to the present invention may be isolated from the aqueous medium following precipitation (or removal of the water-miscible organic solvent, if used). The particles may be separated using conventional techniques, for example by centrifuging, reverse osmosis, membrane filtration, lyophilisation or spray drying. Isolation of the particles is useful when the particles comprise a pharmacologically active compound having a solubility in water at 25°C of less than 0.5 mg/ml because it allows the particles to be washed and re-suspended in a sterile aqueous medium to give a suspension suitable for administration to a warm blooded mammal (especially a human), for example by oral or parenteral (e.g. intravenous) administration.

In this embodiment an agent may be added to the suspension prior to isolation of the particles to prevent agglomeration of the solid particles during isolation (for example spray drying or lyaphilisation). Suitable agents include for example a sugar such as mannitol. Isolation of the particles from the suspension is also useful when it is desirable to store the particles as a powder. The powder may then be re-suspeaded in an aqueous medium prior to use. This is particularly useful for the pharmacologically active substance. The isolated particles of the substance may then be stored as a powder in, for example a vial and subsequently be re-suspended in a suitable liquid medium for administration to a patient as described above.

Alternatively the isolated particles may be used to prepare solid formulations, for example by blending the particles with suitable excipients/carriers and granulating or compressing the resulting mixture to form a tablet or granules suitable for oral administration. Alternatively the particles may be suspended, dispersed or encapsulated in a suitable matrix system, for example a biocompatible polymeric matrix, for example a hydroxypropyl methylcellulose (HPMC) or polylactide/glycloide polymer to give a controlled or sustained release formulation.

In another embodiment of the present invention the process is performed under aseptic conditions, thereby providing a sterile dispersion directly which can be administered to a warm blooded mammal as described above without the need for additional purification or sterilisation steps. Alternatively, the dispersion may be sterile filtered following precipitation and optional removal of the water-miscible organic solvent to leave a sterile suspension.

According to a further aspect of the present invention there is provided a stable aqueous dispersion comprising a continuous aqueous phase in which is dispersed solid particles comprising an inhibitor and a pharmacologically active substance having a solubility in water at 25°C of less than 0,5 mg/ml. wherein said dispersion is obtainable by the process according to the present invention; and wherein:

(i) the inhibitor is a non-polymeric hydrophobic organic compound
(ii) the inhibitor is less soluble in water than the pharmacologically active substance; and
(iii) the inhibitor is not a phospholipid, and
(iv) the inhibitor is sufficiently miscible with the pharmacologically active substance to form solid particles in the dispersion comprising a substantially single phase mixture of the substance and the inhibitor.

The dispersion according to this aspect of the present invention exhibit little or no particle growth upon storage, mediated by Ostwald ripening (i.e. the dispersion is a stable dispersion as defined above in relation to the first aspect of the invention).

The particles preferably have a mean diameter of less than 1$\mu$m and more preferably less than 500nm. It is especially preferred that the particles in the dispersion have a mean particle size of from 10 to 500nm, more especially from 50 to 300nm and still more especially from 100 to 200nm.

The weight fraction of inhibitor in the particles is preferably less than 0.5, more preferably 0.3 or less, for example from 0.05 to 0.3, preferably from 0.06 to 0.25.

In this embodiment it is preferred that the miscibility of the pharmacologically active material and inhibitor are sufficient to give substantially single phase solid particles, more preferably the inhibitor/substance mixture has a $\chi$ value of <2.5, more preferably 2 or less, for example from 0 to 2, preferably from 0.1 to 2, wherein the $\chi$ value is as hereinbefore defined.

The particles may contain a single pharmacologically active substance or two or such substances. The paroles may contain a single inhibitor or a combination of an in inhibitor and one or more co-inhibitors as hereinbefore described.

The dispersions according to the present invention may be administered to a warm blooded mammal (especially a human), for example by oral or parenteral (e.g. intravenous) administration. In an alternative embodiment the dispersion may be used as a granulation liquid in a wet granulation process to prepare granules comprising the pharmacologically active material having a solubility in water at 25°C of less than 0,5 mg/ml and one or more excipients (optionally after first concentrating the dispersion by removal of excess aqueous medium). The resulting granules may then be used directly, for example by filling into capsules to provide a unit dosage containing the granules. Alternatively the granules may be optionally mixed with further excipients, disintegrants, binders, lubricants etc. and compressed into a tablet suitable for oral administration. If required the tablet may be coated to provide control over the release properties of the tablet or to protect it against degradation, for example through exposure to light and/or moisture. Wet granulation techniques and excipients suitable for use in tablet formulations are well known in the art.

According to a further aspect of the present invention there is provided a solid particle comprising an inhibitor and a pharmacologically active substance obtainable by the process according to the present invention, wherein the substance and the inhibitor are as hereinbefore defined in relation to the first aspect of the present invention.

According to a further aspect of the present invention there is provided a solid particle comprising an inhibitor and a pharmacologically active substance obtainable by the process according to the present invention, for use as a medicament, wherein the substance and the inhibitor are as hereinbefore defined in relation to the first aspect of the present invention.

According to a further aspect of the present invention there is provided a pharmaceutical composition comprising a pharmaceutically acceptable carrier or diluent in association with a solid particle comprising an inhibitor and a pharmacologically active substance having a solubility in water at 25°C of less than 0,5 mg/ml obtainable by the process according to the present invention.

Suitable pharmaceutically acceptable carders or diluents are well known excipients used in the preparation of pharmaceutical formulations, for example, fillers, binders, lubricants, disintegrants and/or release controlling/modifying excipients.

According to a further aspect of the present invention there is provided a method for inhibiting Ostwald ripening in a dispersion of solid pharmacologically active particles having a solubility in water as 25°C of less than 0,5 mg/ml in an aqueous medium comprising:

combining (a) a first solution comprising a pharmacologically active substance having a solubility in water at 25°C of less than 0.5 mg/ml, a water-miscible organic solvent and an inhibitor with (b) an aqueous phase comprising water and optionally a stabiliser, thereby precipitating solid particles comprising the inhibitor and the pharmacologically active substance to give a dispersion of the solid pharmacologically active particles in an aqueous medium; and optionally removing the water-miscible organic solvent from the dispersion;

wherein:

(i) the inhibitor is a non-polymenc hydrophobic organic compound
(ii) the inhibitor is less soluble in water than the pharmacologically active substance; and
(iii) the inhibitor is not a phospholipid, and
(iv) the inhibitor is sufficiently miscible with the pharmacologically active substance to form solid particles in the dispersion comprising a substantially single phase mixture of the substance and inhibitor.
Preferred inhibitors and pharmacologically active substances for use in this embodiment are as hereinbefore defined in relation to the first aspect of the present invention.

According to a further aspect of the present invention there is provided the use of an inhibitor to prevent or inhibit Ostwald ripening in a dispersion of solid pharmacologically active having a solubility in water at 25°C of less than 0.5 mg/ml in an aqueous medium wherein:

(i) the inhibitor is a non-polymeric hydrophobic organic compound ;
(ii) the inhibitor is less soluble in water than the pharmacologically active substance having a solubility in water at 25°C of less than 0,5 mg/ml ; and
(iii) the inhibitor is not a phospholipid, and (iv) the inhibitor is sufficiently miscible with the pharmacologically active substance to form solid particles in the dispersion comprising a substantially single phase mixture of the substance and the inhibitor.

Preferred inhibitors and pharmacologically active substances for use in this embodiment are as hereinbefore defined in relation to the first aspect of the present invention.

The invention is further illustrated by the following examples in which all parts are pans by weight unless stated otherwise.

Particle sizes are quoted as the intensity-averaged particle size determined by dynamic light scattering using a Coulter N4MD.

## Brief description of the Drawings

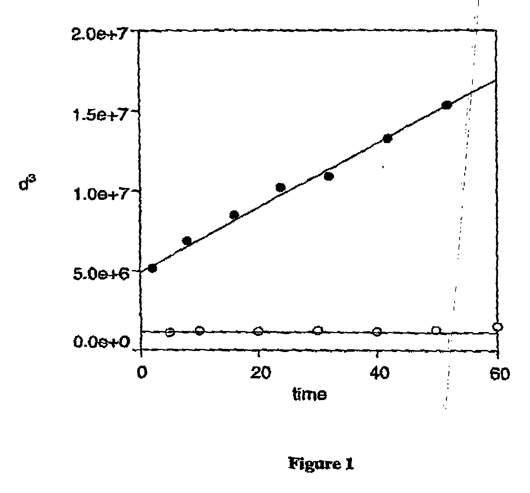

Figure 1 is a graph of the (mean particle diameter)$^3$ (nm$^3$) against time (minutes) for particles of felodipine prepared with and without the use of an inhibitor (Miglyol 812N). The open circles in figure 1 represent the felodipine particles prepared with the inhibitor (Miglyol 812N) and the solid circles felodipine particles prepared without an inhibitor. Figure 1 clearly shows that the presence of the inhibitor eliminated Ostwald ripening in the felodipine particles and the particle size remains constant. Whereas the felodipine particles prepared without an inhibitor grew rapidly with time.

## Example 1 :

### Felodipine/Miglyol 812 N (4:1 w/w) Dispersion

A solution of 91 mM Felodipine and 8.7 mg/ml Miglyol 812N in dimethylacetamide (DMA) was prepared. 0.01 ml of this solution was added rapidly to 0.9 ml of an aqueous solution containing 0.2%w/w polyvinylpyrrolidone (PVP) and 0.25 mM sodium dodecyl sulfate (SDS). The aqueous solution was sonicated during the addition of the organic solution using an ultrasonic bath. This resulted in the precipitation of particles with a mean size of 100 nm, as measured by dynamic light scattering using a Coulter N4MD. No increase in particle size was observed over a period of 2 hours, at 20°C. The Felodipine/inhibitor $\chi$ parameter was calculated to be 0.4 using Equation I described herein.

$T_m$ and $\Delta S_m$ were determined by DSC analysis on a sample of crystalline felodigine using a Mettler-Toledo DSC 820 using and open vial configuration and a scanning speed of 10 K/min to give the entropy of melting, $\Delta S_m$=72 J/mol,K and melting point $T_m$=417 K

The mole-fraction solubility of the felodipine/Miglyol 812N ($x^s_1$ in Equation I) was determined by magnetically stirring an excess of crystalline felodipine (approx 2-5 times that required for a saturated solution) in Miglyol 812N (5-25 ml) at 350 rpm (protected from light and sealed under a nitrogen atmosphere) for 2 days at room temperature. The resulting mixture was filtered to remove solids (0.2$\mu$m filter) and then analyzed using HPLC to determine the quantity of felodipine dissolved in the Miglyol 812N. The solubility of felodipine in Miglyol 812N was 69mM giving a mole fraction solubility of 0.069/1.9=0.036, where 1.9 is the apparent molarity of Miglyol 812N. Miglyol 812N is a mixture of approximately 60 % C8-triglyceride (Mw 471) and 40 % C10-triglyceride (Mw 555) and has a density of approximately 0.945 g/cm$^3$. Thus, the apparent molarity of Miglyol 812N is 945/(0.6*471+0.4*555)=1.9].

### Comparative Example 1

Example 1 was repeated but without using Miglyol 812N. The process produced particles mean particle diameter of approximately 170nm. The particle size increased rapidly over a period of 1 hour at 20°C from 170 to 250 nm and after 2 hours had increased to 370nm.

Figure 1 shows the cube of the average particle diameter against time for the particles prepared according to Example 1 (with an inhibitor) and those prepared according to the comparative example (no inhibitor). It is clear from Figure 1 that the dispersion according to the present invention shows no particle size increase whereas the dispersion prepared without the use of an inhibitor shows a rapid increase in particle size as a result of Ostwald ripening.

### Example 2: Felodipine/Miglyol 812 N (10:1 w/w) Dispersion

A solution of 100 mM Felodipine and 3.85 mg/ml Miglyol 812 N in dimethylacetamide (DMA) was prepared. 0.01 ml of this solution was added rapidly to 0.99 ml of an aqueous solution containing 0.2%w/w polyvinylpyrrolidone (PVP) and 0.25 mM sodium dodecyl sulfate (SDS), as described in Example 1. This resulted in the precipitation of particles with a mean size of 1.20 nm. No further growth was observed after 1 hour at 20°C. The Felodipine/mhibitor x parameter was calculated to be 0.4 using the method described in Example 1.

**Comparative Example 2**

Example 2 was repeated but without using the inhibitor (Miglyol 812 N). The particle size increased rapidly over a period of 1 hour at 20°C from 170 to 250 nm, and after 2 hours the size was 370 nm.

**Example 3:Felodipine/Trilaurin (8:1 w/w) Dispersion**

A solution of 100 mM Felodipine and 4.8 mg/ml Trilaurin in dimethylacetamide (DMA) was prepared. 0.01 ml of this solution was added rapidly to 0.99 ml of an aqueous solution containing 0.2%w/w polyvinylpyrrolidone (PVP) and 0.25 mM sodium dodecyl sulfate (SDS), as described in Example 1. This resulted in the precipitation of particles with a mean size of 160 nm. No further growth was observed after 1 hour at 20° C.

**Comparative Example 3**

Example 3 was repeated but without using the inhibitor (Trilaurin). The particle size increased rapidly over a period of 1 hour at 20°C from 170 to 250 nm, and after 2 hours the size was 370 nm.

**Example 4 :Bicalutamide/Miglyol 812 N (4:1 w/w) Dispersion**

A solution of 100 mM bicalutamide and 10.8 mg/ml Miglyol 812 N in dimethylacetamide (DMA) was prepared. 0.01 ml of this solution was added rapidly to 0.99 ml of an aqueous solution containing 0.2%w/w polyvinylpyrrolidone (PVP), as described in Example 1. This resulted in the precipitation of particles with a mean size of 270 nm. No Ostwald ripening was observed after 1 hour at 20°C. The bicalutamide/inhibitor $\chi$ parameter was calculated to be 1.4 using the method described in Example 1.

**Comparative Example 4**

Example 4 was repeated but without using the inhibitor (Miglyol 812 N). The particle size increased rapidly over a period of 20 minutes at 20°C from 210 to 700 nm.

**Example 5:Nifedipine/Miglyol 812N (4:1 w/w)Dispersion**

A solution of 100 mM Nifedipine and 8.6 mg/ml Miglyol 812 N in dimethylacetamide (DMA) was prepared. 0.055 ml of this solution was added rapidly to 0.945 ml of an aqueous solution containing 0.2%w/w polyvinylpyrrolidone (PVP) and 0.25 mM sodium dodecyl sulfate (SDS), as described in Example 1. This resulted in the precipitation of particles with a mean size of 120 nm and no further growth was observed after 1 hour at 20 °C. The Nifedipine/inhibitor $\chi$ parameter was determined to be1.2 using the method described in Example 1.

**Comparative Example 5**

Example 5 was repeated but without using the inhibitor (Miglyol 812 N). The particle size increased rapidly over a period of 60 minutes at 20°C from 220 to 1100 nm.

**Example 6**

**8-[(2-ethyl-6-methylbenzyl)amino]-2,3-dimethylimidazo[1,2-*a*]pyridine-6-carboxamide /Miglyol 812 N/1-decanol (8:1:1 w/w) Dispersion**

A solution of 100 mM 8-[(2-ethyl-6-methylbenzyl)amino]-2,3-dimethylimidazo[1,2-*a*]pyridine-6-carboxamide (described in WO99/55706), 4.2 mg/ml Miglyol 812 N (inhibitor) and 4.2 mg/ml 1-decanol (co-inhibitor) in dimethylacetamide (DMA) was prepared. 0.01 ml of this solution was added rapidly to 0.99 ml of an aqueous solution containing 0.2%w/w polyvinylpyrrolidone (FVP) and 0.25 mM sodium dodecyl sulfate (SDS), as described in Example 1. This resulted in the precipitation of particles with a mean size of 220 nm and no further growth was observed after 1 hour at 20 °C. The drug/inhibitor $\chi$ parameter was determined to be 0.6 using the method described in Example 1, by measuring the solubility of the compound in a 1:1 by weight mixture of the Miglyol 812N and 1-decanol. In this system $\Delta S_m$=66 J/mol,K, $T_m$=491 K, the solubility of the substance in the Miglyol 812N/l-decanol mixture was 37mM, the mole fraction solubility =0.037/3,6=0.0103 where 3.6 is the apparent molarity of the 1:1 Miglyo1812N and 1-decanol mixture.

In another experiment where 1-decanol was replaced with Miglyol 812 N the particle size increased slowly over a period of 100 minutes at 20°C from 210 to 280 nm. The drug/inhibitor $\chi$ parameter for this latter system was determined to 2.8 as described in Example 1 ($\Delta S_m$=66 J/mol,K, $T_m$=491 K, the solubility of the substance in the Miglyol was 2.2mM and the mole fraction solubility was 0.0022/1.9=0.00116 where 1.9 is the apparent molarity of Miglyol 812N).

This example illustrates that for the preferred inhibitor ($\chi$ parameter <2.5) Ostwald ripening is eliminated whilst for those systems in which the $\chi$ parameter is higher Ostwald ripening is reduced but may not be eliminated completely.

## Claims

1. A process for the preparation of a stable dispersion of solid particles in an aqueous medium comprising:

   combining (a) a first solution comprising a pharmacologically active substance having a solubility in water at 25°C of less than 0.5 mg/ml, a water-miscible organic solvent and an inhibitor, with (b) an aqueous phase comprising water and optionally a stabiliser, thereby precipitating solid particles comprising the inhibitor and the pharmacologically active substance; and optionally removing the water-miscible organic solvent;

   wherein:

   (i) the inhibitor is a non-polymeric hydrophobic organic compound;
   (ii) the inhibitor is less soluble in water than the pharmacologically active substance;
   (iii) the inhibitor is not a phospholipid; and
   (iv) the inhibitor is sufficiently miscible with the pharmacologically active substance to form solid particles in the dispersion comprising a substantially single phase mixture of the substance and the inhibitor.

2. A process according to claim 1 wherein the inhibitor is a mixture of triglycerides obtainable by esterifying glycerol with a mixture of medium chain fatty acids.

3. A process according to claim 2 wherein the inhibitor is a mixture of triglycerides containing acyl groups with from 8 to 12 carbon atoms.

4. A process according to any one of the preceding claims wherein the inhibitor further comprises a co-inhibitor selected from a long chain aliphatic alcohol containing 6 or more carbon atoms.

5. A process according to any one of the preceding claims wherein the miscibility of the inhibitor and pharmacologically active substance having a solubility in water at 25°C of less than 0.5 mg/ml is sufficient to give an interaction parameter, $\chi$, of less than 2.5.

6. A process according to any one of the preceding claims wherein the aqueous phase contains a stabiliser.

7. A process according to claim 6 wherein the stabiliser comprises a polymeric dispersant and a surfactant.

8. A process according to claim 1 for the preparation of a stable dispersion, of solid particles of a pharmacologically active substance having a solubility in water at 25°C of less than 0.5 mg/ml, in an aqueous medium comprising:

   combining (a) a first solution comprising the pharmacologically active substance, a water-miscible organic solvent and an inhibitor with (b) an aqueous phase comprising water and optionally a stabiliser, thereby precipitating solid particles comprising the inhibitor and the pharmacologically active substance; and optionally removing the water-miscible organic solvent;

   wherein the inhibitor is less soluble in water than the pharmacologically active substance, which inhibitor is selected from one or more of:

   (i) a mono-, di- or a tri-glyceride of a fatty acid;
   (ii) a fatty acid mono- or di-ester of a $C_{2-10}$ diol;
   (iii) a fatty acid ester of an alkanol or a cycloalkanol;
   (iv) a wax;
   (v) a long chain aliphatic alcohol; and
   (vi) a hydrogenated vegetable oil.

9.  A process according to any one of the preceding claims wherein the mean particle size of the solid particles is less than 1μm.

10. A process according to any one of the preceding claims further comprising the step of isolating the solid particles from the dispersion.

11. A stable aqueous dispersion comprising a continuous aqueous phase in which is dispersed solid particles comprising an inhibitor and a pharmacologically active substance having a solubility in water at 25°C of less than 0.5 mg/ml, obtainable by the process according to any one of the preceding claims wherein:

    (i) the inhibitor is a non-polymeric hydrophobic organic compound;
    (ii) the inhibitor is less soluble in water than the pharmacologically active substance;
    (iii) the inhibitor is not a phospholipid; and
    (iv) the inhibitor is sufficiently miscible with the pharmacologically active substance to form solid particles in the dispersion comprising a substantially single phase mixture of the substance and the inhibitor.

12. A solid particle comprising an inhibitor and a pharmacologically active substance having a solubility in water at 25°C of less than 0.5 mg/ml, obtainable by the process according to any one of claims 1 to 10.

13. A solid particle according to claim 12 for use as a medicament.

14. A pharmaceutical composition comprising a solid particle according to claim 12 in association with a pharmaceutically acceptable carrier or diluent.

15. A method for inhibiting Ostwald ripening in a dispersion of solid pharmacologically active particles having a solubility in water at 25°C of less than 0.5 mg/ml, in an aqueous medium comprising:

    combining (a) a first solution comprising a pharmacologically active substance having a solubility in water at 25°C of less than 0.5 mg/ml, a water-miscible organic solvent and an inhibitor with (b) an aqueous phase comprising water and optionally a stabiliser, thereby precipitating solid particles comprising the inhibitor and the pharmacologically active substance to give a dispersion of the solid pharmacologically active particles in an aqueous medium; and optionally removing the water-miscible organic solvent from the dispersion;

    wherein:

    (i) the inhibitor is a non-polymeric hydrophobic organic compound;
    (ii) the inhibitor is less soluble in water than the pharmacologically active substance; and
    (iii) the inhibitor is not a phospholipid; and
    (iv) the inhibitor is sufficiently miscible with the pharmacologically active substance to form solid particles in the dispersion comprising a substantially single phase mixture of the substance and the inhibitor.

16. Use of an inhibitor to prevent or inhibit Ostwald ripening in a dispersion of solid pharmacologically active particles having a solubility in water at 25°C of less than 0.5 mg/ml, in an aqueous medium wherein:

    (i) the inhibitor is a non-polymeric hydrophobic organic compound;
    (ii) the inhibitor is less soluble in water than the pharmacologically active substance having a solubility in water at 25°C of less than 0.5 mg/ml; and
    (iii) the inhibitor is not a phospholipid; and
    (iv) the inhibitor is sufficiently miscible with the pharmacologically active substance to form solid particles in the dispersion comprising a substantially single phase mixture of the substance and the inhibitor.

17. Any of claims 1-16 wherein the pharmacologically active substance has a solubility in water at 25°C of less than 0.1 mg/ml.

18. Any of claims 1-16 wherein the pharmacologically active substance has a solubility in water at 25°C of less than 0.05 mg/ml.

**EP 1 416 917 B1**

**Patentansprüche**

1. Verfahren zur Herstellung einer stabilen Dispersion von festen Teilchen in einem wässrigen Medium, umfassend:

   das Kombinieren (a) einer ersten Lösung, die eine pharmakologisch aktive Substanz mit einer Löslichkeit in Wasser bei 25 ºC von weniger als 0,5 mg/ml, ein in Wasser mischbares organisches Lösungsmittel und einen Inhibitor umfasst, mit (b) einer wässrigen Phase, die Wasser und gegebenenfalls einen Stabilisator umfasst, wodurch feste Teilchen präzipitieren, welche den Inhibitor und die pharmakologisch aktive Substanz umfassen; und gegebenenfalls das Entfernen des in Wasser mischbaren organischen Lösungsmittels;

   worin:

   (i) der Inhibitor eine nicht-polymere hydrophobe organische Verbindung ist;
   (ii) der Inhibitor in Wasser weniger löslich als die pharmakologisch aktive Substanz ist;
   (iii) der Inhibitor nicht ein Phospholipid ist; und
   (iv) der Inhibitor ausreichend mit der pharmakologisch aktiven Substanz mischbar ist, um feste Teilchen in der Dispersion zu bilden, umfassend eine im Wesentlichen einzelphasige Mischung aus der Substanz und dem Inhibitor.

2. Verfahren gemäß Anspruch 1, wobei der Inhibitor eine Mischung aus Triglyceriden ist, erhältlich durch Verestern von Glycerol mit einer Mischung von Fettsäuren mit mittlerer Kettenlänge.

3. Verfahren gemäß Anspruch 2, wobei der Inhibitor eine Mischung von Triglyceriden, enthaltend Acylgruppen mit 8 bis 12 Kohlenstoffatomen, ist.

4. Verfahren gemäß mindestens einem der vorhergehenden Ansprüche, wobei der Inhibitor ferner einen Co-Inhibitor umfasst, der aus einem langkettigen aliphatischen Alkohol, welcher 6 oder mehr Kohlenstoffatome enthält, gewählt ist.

5. Verfahren gemäß mindestens einem der vorhergehenden Ansprüche, wobei die Mischbarkeit des Inhibitors und der pharmakologisch aktiven Substanz mit einer Löslichkeit in Wasser bei 25ºC von weniger als 0,5 mg/ml ausreichend ist, um einen Wechselwirkungsparameter, $\chi$, von weniger als 2,5 zu erhalten.

6. Verfahren gemäß mindestens einem der vorhergehenden Ansprüche, wobei die wässrige Phase einen Stabilisator enthält.

7. Verfahren gemäß Anspruch 6 wobei der Stabilisator ein polymeres Dispergiermittel und ein Surfactant umfasst.

8. Verfahren gemäß Anspruch 1 zur Herstellung einer stabilen Dispersion von festen Teilchen einer pharmakologisch aktiven Substanz mit einer Löslichkeit in Wasser bei 25ºC von weniger als 0,5 mg/ml in einem wässrigen Medium, umfassend:

   das Kombinieren (a) einer ersten Lösung, die die pharmakologisch aktive Substanz, ein mit Wasser mischbares organisches Lösungsmittel und einen Inhibitor umfasst, mit (b) einer wässrigen Phase, die Wasser und gegebenenfalls einen Stabilisator umfasst, wodurch feste Teilchen präzipitiert werden, die den Inhibitor und die pharmakologisch aktive Substanz umfassen; und gegebenenfalls das Entfernen des mit Wasser mischbaren organischen Lösungsmittels:

   wobei der Inhibitor in Wasser weniger löslich als die pharmakologisch aktive Substanz ist, wobei der Inhibitor aus einem oder mehreren von folgenden gewählt wird:

   (i) einem Mono-, Di- oder einem Tri-Glycerid einer Fettsäure;
   (ii) einem Fettsäuremono- oder -di-ester eines C2-10-Diols;
   (iii) einem Fettsäureester eines Alkanols oder eines Cycloalkanols;
   (iv) einem Wachs;
   (v) einem langkettigen aliphatischen Alkohol; und
   (vi) einem hydrierten Pflanzenöl.

9.  Verfahren gemäß mindestens einem der vorhergehenden Ansprüche, wobei die mittlere Teilchengröße der festen Teilchen geringer als 1 μm ist.

10. Verfahren gemäß mindestens einem der vorhergehenden Ansprüche, ferner umfassend den Schritt des Isolierens der festen Teilchen von der Dispersion.

11. Stabile wässrige Dispersion, umfassend eine kontinuierliche wässrige Phase, in welcher feste Teilchen dispergiert sind, umfassend einen Inhibitor und eine pharmakologisch aktive Substanz mit einer Löslichkeit in Wasser bei 25°C von weniger als 0,5 mg/ml, erhältlich durch das Verfahren gemäß mindestens einem der vorhergehenden Ansprüche, worin:

    (i) der Inhibitor eine nicht-polymere hydrophobe organische Verbindung ist;
    (ii) der Inhibitor in Wasser weniger löslich als die pharmakologisch aktive Substanz ist;
    (iii) der Inhibitor nicht ein Phospholipid ist; und
    (iv) der Inhibitor ausreichend mit der pharmakologisch aktiven Substanz mischbar ist, um feste Teilchen in der Dispersion zu bilden, umfassend eine im Wesentlichen einzelphasige Mischung aus der Substanz und dem Inhibitor.

12. Festes Teilchen, umfassend einen Inhibitor und eine pharmakologisch aktive Substanz mit einer Löslichkeit in Wasser bei 25°C von weniger als 0,5 mg/ml, erhältlich durch das Verfahren gemäß mindestens einem der Ansprüche 1 bis 10.

13. Festes Teilchen gemäß Anspruch 12 zur Verwendung als ein Medikament.

14. Pharmazeutische Zusammensetzung, umfassend ein festes Teilchen gemäß Anspruch 12 in Assoziation mit einem pharmazeutisch annehmbaren Träger oder Verdünnungsmittel.

15. Verfahren zur Inhibierung der Ostwald-Reifung in einer Dispersion von festen, pharmakologisch aktiven Teilchen mit einer Löslichkeit in Wasser bei 25°C von weniger als 0,5 mg/ml in einem wässrigen Medium, umfassend:

    das Kombinieren (a) einer ersten Lösung, die eine pharmakologisch aktive Substanz mit einer Löslichkeit in Wasser bei 25°C von weniger als 0,5 mg/ml, ein mit Wasser mischbares organisches Lösungsmittel und einen Inhibitor umfasst, mit (b) einer wässrigen Phase, die Wasser und gegebenenfalls einen Stabilisator umfasst, wodurch feste Teilchen präzipitiert werden, die den Inhibitor und die pharmakologisch aktive Substanz umfassen, um eine Dispersion der festen, pharmakologisch aktiven Teilchen in einem wässrigen Medium zu erhalten; und gegebenenfalls das Entfernen des mit Wasser mischbaren organischen Lösungsmittels von der Dispersion;

    worin

    (i) der Inhibitor eine nicht-polymere hydrophobe organische Verbindung ist;
    (ii) der Inhibitor in Wasser weniger löslich als die pharmakologisch aktive Substanz ist;
    (iii) der Inhibitor nicht ein Phospholipid ist; und
    (iv) der Inhibitor ausreichend mit der pharmakologisch aktiven Substanz mischbar ist, um feste Teilchen in der Dispersion zu bilden, umfassend eine im Wesentlichen einzelphasige Mischung aus der Substanz und dem Inhibitor.

16. Verwendung eines Inhibitors, um die Ostwald-Reifung in einer Dispersion von festen, pharmakologisch aktiven Teilchen mit einer Löslichkeit in Wasser bei 25°C von weniger als 0,5 mg/ml in einem wässrigen Medium zu verhindern oder zu inhibieren, worin:

    (i) der Inhibitor eine nicht-polymere hydrophobe organische Verbindung ist;
    (ii) der Inhibitor in Wasser weniger löslich als die pharmakologisch aktive Substanz mit einer Löslichkeit in Wasser bei 25°C von weniger als 0,5 mg/ml ist; und
    (iii) der Inhibitor nicht ein Phospholipid ist; und
    (iv) der Inhibitor ausreichend mit der pharmakologisch aktiven Substanz mischbar ist, um feste Teilchen in der Dispersion zu bilden, umfassend eine im Wesentlichen einzelphasige Mischung aus der Substanz und dem Inhibitor.

**17.** Jedweder der Ansprüche 1 - 16, worin die pharmakologisch aktive Substanz eine Löslichkeit in Wasser bei 25ºC von weniger als 0,1 mg/ml besitzt.

**18.** Jedweder der Ansprüche 1 - 16, worin die pharmakologisch aktive Substanz eine Löslichkeit in Wasser bei 25ºC von weniger als 0,05 mg/ml besitzt.

**Revendications**

**1.** Procédé pour la préparation d'une dispersion stable de particules solides dans un milieu aqueux comprenant :

la combinaison de (a) une première solution comprenant un principe actif pharmacologique de solubilité dans l'eau à 25 °C inférieure à 0,5 mg/mL, un solvant organique miscible à l'eau et un inhibiteur, et de (b) une phase aqueuse comprenant de l'eau et, éventuellement, un agent stabilisant, ladite combinaison aboutissant à la précipitation de particules solides comprenant l'inhibiteur et le principe actif pharmacologique ; et éventuellement l'élimination du solvant organique miscible à l'eau ;

où :

(i) l'inhibiteur est un composé organique hydrophobe non polymère ;
(ii) l'inhibiteur est moins soluble dans l'eau que le principe actif pharmacologique ;
(iii) l'inhibiteur n'est pas un phospholipide ; et
(iv) l'inhibiteur est suffisamment miscible au principe actif pharmacologique pour former des particules solides dans la dispersion comprenant un mélange essentiellement monophasique du principe et de l'inhibiteur.

**2.** Procédé selon la revendication 1 où l'inhibiteur est un mélange de triglycérides pouvant être obtenu par estérification du glycérol à l'aide d'un mélange d'acides gras à chaînes de longueur moyenne.

**3.** Procédé selon la revendication 2 où l'inhibiteur est un mélange de triglycérides contenant des groupements acyle comportant entre 8 et 12 atomes de carbone.

**4.** Procédé selon l'une quelconque des revendications précédentes où l'inhibiteur comprend en outre un co-inhibiteur sélectionné parmi un alcool aliphatique à longue chaîne comprenant 6 atomes de carbone ou plus.

**5.** Procédé selon l'une quelconque des revendications précédentes où la miscibilité de l'inhibiteur et du principe actif pharmaceutique dont la solubilité dans l'eau à 25 °C est inférieure à 0,5 mg/mL est suffisante pour que le paramètre d'interaction $\chi$ soit inférieur à 2,5.

**6.** Procédé selon l'une quelconque des revendications précédentes où la phase aqueuse contient un agent stabilisant.

**7.** Procédé selon la revendication 6 où l'agent stabilisant comprend un agent dispersant polymère et un tensioactif.

**8.** Procédé selon la revendication 1 pour la préparation d'une dispersion stable de particules solides d'un principe actif pharmacologique de solubilité dans l'eau à 25 °C inférieure à 0,5 mg/mL, dans un milieu aqueux, comprenant :

la combinaison de (a) une première solution comprenant le principe actif pharmacologique, un solvant organique miscible à l'eau et un inhibiteur, et de (b) une phase aqueuse comprenant de l'eau et, éventuellement, un agent stabilisant, ladite combinaison aboutissant à la précipitation de particules solides comprenant l'inhibiteur et le principe actif pharmacologique ; et éventuellement l'élimination du solvant organique miscible à l'eau ;

où l'inhibiteur est moins soluble dans l'eau que le principe actif pharmacologique, ledit inhibiteur étant sélectionné parmi une ou plusieurs des substances suivantes :

(i) un mono-, di-, ou tri-glycéride, d'un acide gras ;
(ii) un mono- ou di-ester d'acide gras d'un diol en $C_2$-$C_{10}$ ;
(iii) un ester d'acide gras d'un alcanol ou d'un cycloalcanol ;
(iv) une cire ;
(v) un alcool aliphatique à longue chaîne ; et

(vi) une huile végétale hydrogénée.

9. Procédé selon l'une quelconque des revendications précédentes où la granulométrie moyenne des particules solides est inférieure à 1 $\mu$m.

10. Procédé selon l'une quelconque des revendications précédentes et comprenant en outre l'étape d'isolement des particules solides de la dispersion.

11. Dispersion aqueuse stable comprenant une phase aqueuse continue dans laquelle sont dispersées des particules solides comprenant un inhibiteur et un principe actif pharmacologique de solubilité dans l'eau à 25 °C inférieure à 0,5 mg/mL, pouvant être obtenue par le procédé selon l'une quelconque des revendications précédentes où :

   (i) l'inhibiteur est un composé organique hydrophobe non polymère ;
   (ii) l'inhibiteur est moins soluble dans l'eau que le principe actif pharmacologique ;
   (iii) l'inhibiteur n'est pas un phospholipide ; et
   (iv) l'inhibiteur est suffisamment miscible au principe actif pharmacologique pour former des particules solides dans la dispersion comprenant un mélange essentiellement monophasique du principe et de l'inhibiteur.

12. Particule solide comprenant un inhibiteur et un principe actif pharmacologique de solubilité dans l'eau à 25 °C inférieure à 0,5 mg/mL, pouvant être obtenue par le procédé selon l'une quelconque des revendications 1 à 10.

13. Particule solide selon la revendication 12 pouvant être employée en tant que médicament.

14. Composition pharmaceutique comprenant une particule solide selon la revendication 12 associée à un vecteur ou un diluant de qualité pharmaceutique.

15. Méthode d'inhibition du mûrissement d'Ostwald au sein d'une dispersion dans un milieu aqueux de particules solides actives sur le plan pharmacologique de solubilité dans l'eau à 25 °C inférieure à 0,5 mg/mL, comprenant :

   la combinaison de (a) une première solution comprenant un principe actif pharmacologique de solubilité dans l'eau à 25 °C inférieure à 0,5 mg/mL, un solvant organique miscible à l'eau et un inhibiteur, et de (b) une phase aqueuse comprenant de l'eau et, éventuellement, un agent stabilisant, ladite combinaison aboutissant à la précipitation de particules solides comprenant l'inhibiteur et le principe actif pharmacologique ce qui permet d'obtenir une dispersion des particules solides actives sur le plan pharmacologique dans un milieu aqueux ; et éventuellement l'élimination du solvant organique miscible à l'eau de la dispersion ;

   où :

   (i) l'inhibiteur est un composé organique hydrophobe non polymère ;
   (ii) l'inhibiteur est moins soluble dans l'eau que le principe actif pharmacologique ; et
   (iii) l'inhibiteur n'est pas un phospholipide ; et
   (iv) l'inhibiteur est suffisamment miscible au principe actif pharmacologique pour former des particules solides dans la dispersion comprenant un mélange essentiellement monophasique du principe et de l'inhibiteur.

16. Utilisation d'un inhibiteur pour empêcher ou inhiber le mûrissement d'Ostwald au sein d'une dispersion dans un milieu aqueux de particules solides actives sur le plan pharmacologique de solubilité dans l'eau à 25 °C inférieure à 0,5 mg/mL, où :

   (i) l'inhibiteur est un composé organique hydrophobe non polymère ;
   (ii) l'inhibiteur est moins soluble dans l'eau que le principe actif pharmacologique de solubilité dans l'eau à 25 °C inférieure à 0,5 mg/mL ; et
   (iii) l'inhibiteur n'est pas un phospholipide ; et
   (iv) l'inhibiteur est suffisamment miscible au principe actif pharmacologique pour former des particules solides dans la dispersion comprenant un mélange essentiellement monophasique du principe et de l'inhibiteur.

17. L'une quelconque des revendications 1 à 16 où le principe actif pharmacologique a une solubilité dans l'eau à 25 °C inférieure à 0,1 mg/mL.

**18.** L'une quelconque des revendications 1 à 16 où le principe actif pharmacologique a une solubilité dans l'eau à 25 °C inférieure à 0,05 mg/mL.

**Figure 1**

21

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5145648 A
- US 4826689 A
- US 4997454 A
- US 6074986 A
- WO 9507614 A
- EP 589838 A
- US 4348385 A
- WO 9904766 A
- US 5100591 A
- US 4610868 A
- WO 9955706 A

**Non-patent literature cited in the description**

- **OSTWALD.** *Z Phys. Chem.,* 1900, vol. 34, 495-503
- **W. J. HIGUCHI ; J. MISRA.** *J. Pharm. Sci.,* 1962, vol. 51, 459
- **WELIN-BERGER et al.** *Int. Jour. of Pharmaceutics,* 2000, vol. 200, 249-260
- **JÖNSSON, B. ; LINDMAN, K. ; HOLMBERG, B. ; KRONBERG.** Surfactants and Polymers in Solution. John Wiley & Sons, 1998
- **NEAU et al.** *Pharmaceutical Research,* 1997, vol. 14, 601